# EUROPÄISCHE PATENTSCHRIFT

(11) **EP 0 403 889 B1**
(45) Veröffentlichungstag und Bekanntmachung des Hinweises auf die Patenterteilung: **02.02.1994**
(21) Anmeldenummer: 90110851.4
(22) Anmeldetag: 08.06.1990
(51) Int. Cl.: C07D 249/12

(54) **Verfahren zur Herstellung von 4-Amino-1,2,4-triazol-5-onen**
Method for the preparation of 4-amino-1,2,4-triazol-5-ones
Procédé de préparation d'amino-4 triazole-1,2,4 ones-5

(30) Priorität: 21.06.1989 DE 3920270
(43) Veröffentlichungstag der Anmeldung: 27.12.1990
(73) Patentinhaber: BAYER AG, 51368 Leverkusen (DE)
(72) Erfinder: König, Klaus, Dr., D-5068 Odenthal (DE); Müller, Klaus-Helmut, Dr., D-4000 Düsseldorf 13 (DE); Rohe, Lothar, Dr., D-5600 Wuppertal 1 (DE)

(56) Entgegenhaltungen:
- EP-A- 0 321 833
- CHEMICAL ABSTRACTS, Band 106, Nr. 17, 27. April 1987, Columbus, Ohio, USA; IKIZLER A. et al.: "1,2,4-Triazolin-5-ones and hydroxamic acid derivatives", Seite 682, Spalte 1, Zusammenfassung-Nr. 138 338e
- CHEMICAL ABSTRACTS, Band 90, Nr. 25, 18. Juni 1979, Columbus, Ohio, USA; MILCENT R. et al.: "Synthesis of 4-amino-3-aryl-1,2,4-triazol-5-ones", Seite 617, Spalte 1, Zusammenfassung-Nr. 203 981b
- CHEMICAL ABSTRACTS, Band 83, Nr. 25, 22. Dezember 1975, Columbus, Ohio, USA; ESMAIL R. et al.: "Heterocyclic compounds from urea derivatives. XXII. Thiobenzoylated carbonohydrazides and their cyclization", Seite 346, Spalte 1, Zusammenfassung-Nr. 205 712u
- CHEMICAL ABSTRACTS, Band 70, Nr. 3, 20. Januar 1969, Columbus, Ohio, USA; ROMANOVA I.B. et al.: "Reaction of hydrazine hydrate with carbon monoxide", Seite 282, Spalte 1, Zusammenfassung-Nr. 11 643h
- CHEMICAL ABSTRACTS, Band 62, Nr. 12, 7. Juni 1965, Columbus, Ohio, USA; GEHLEN H. et al.: "1,2,4-Triazol-5-ones. V. Effect of substituents on the rate of hydrolysis in half-concentrated sulfuric acid", Zusammenfassung-Nr. 14 437c
- CHEMICAL ABSTRACTS, Band 63, Nr. 12, 6. Dezember 1965, Columbus, Ohio, USA; KROEGER C.F. et al.: "1,2,4-Triazoles. IX. Syntheses and reactions of 4-amino-1,2,4-triazol-5-ones", Zusammenfassung-Nr. 16 339d
- BEILSTEINS HANDBUCH DER ORGANISCHEN CHEMIE, 4. Auflage, Band 26, 1937, Berlin, Seite 142
- BEILSTEINS HANDBUCH DER ORGANISCHEN CHEMIE, 4. Auflage, Band 26, 1. Teil, 1982, Berlin, Seite 423

## Beschreibung

Die Erfindung betrifft ein neues Verfahren zur Herstellung von 4-Amino-1,2,4-triazol-5-onen, welche Zwischenprodukte zur Herstellung von herbiziden Wirkstoffen sind.

Es ist bekannt, daß man 4-Amino-1,2,4-triazol-5-one erhält, wenn man Carbohydrazid mit Carbonsäuren thermisch cyclisiert (vgl. Chem. Ber 98, 3025 [1965]). Der Nachteil dieses Verfahrens liegt darin, daß der Ringschluß erst nach längerer Reaktionszeit (10 Stunden) erfolgt und unter diesen Reaktionsbedingungen bereits Selbstkondensation des Carbohydrazids zum 4-Aminourazol erfolgt. Die Ausbeuten an gewünschtem Triazolon sind bei diesem Verfahren gering (16 % bis 22 %).

Weiterhin ist bekannt, daß man 4-Amino-1,2,4-triazol-5-one erhält, wenn man 1,5-Diacyl-carbohydrazide in Gegenwart von wäßrigem Alkali cyclisiert. Die hierfür erforderlichen 1,5-Diacylcarbohydrazide werden zuvor durch Erwärmen von Carbohydrazid mit Carbonsäuren hergestellt (vgl. Chem. Ber. 98, 3025 [1965]). Auch bei diesem Verfahren sind die Ausbeuten über beide Stufen nicht befriedigend.

Weiterhin ist bekannt, daß man 4-Amino-1,2,4-triazol-5-one erhält, wenn man N^{β}-Acylcarbazinsäureester mit Hydrazinhydrat umsetzt (vgl. Chem. Ber 98, 3025 [1965]). Nachteilig bei diesem Verfahren sind ebenfalls die geringen Ausbeuten, insbesondere wenn man berücksichtigt, daß die N^{ß}-Acylcarbazinsäureester, welche als Ausgangsverbindungen benotigt werden, ebenfalls erst hergestellt werden müssen. Ein weiterer gravierender Nachteil dieser Methode besteht darin, daß diese Methode nur bestimmte (3-Methyl)-4-Amino-1,2,4-triazol-5-one zugänglich macht. Bereits mit der homologen Ethyl-Verbindung N^{ß}-Propionyl-carbazinsäureethylester findet unter gleichen Bedingungen keine Cyclisierung mehr statt.

Weiterhin ist bekannt, daß man 4-Amino-1,2,4-triazol-5-one der unten beschriebenen Formel (I) erhält, wenn man Carbohydrazid mit Orthoestern thermisch cyclisiert (vgl. Chem. Ber. 27, 55 [1894]; J. Prakt. Chem [2] 52, 454 [1895]; Chem. Ber. 57, 1321 [1924]; Liebigs Ann Chem. 475, 120 [1929] und Inorganic Synthesis 4, 32 [1953]).

Nachteilig bei diesem Verfahren ist, daß als Ausgangsverbindungen Orthoester erforderlich sind, welche selbst in mehrstufiger - über hydrolyseanfällige Zwischenstufen verlaufender - Synthese hergestellt werden müssen, was die Gesamtsynthese aus Kosten- und Umweltgründen wenig vorteilhaft macht.

Ein weiteres bekanntes Verfahren zur Herstellung von 4-Amino-1,2,4-triazol-5-onen besteht in der Umsetzung von Pinner-Salzen mit Carbazinsäureestern und der anschließenden Cyclisierung mit Hydrazinhydrat (vgl. Bull. Soc. Chim. France 1962, 1364; Eur. J. Med. Chem. - Chim. Ther. 1983, 215; Chimica Acta Turcica 7, 269 [1979]). Nachteilig bei diesem Verfahren ist die niedrige Gesamtausbeute und die Herstellung der Pinner-Salze, wozu Reaktionszeiten von mehreren Tagen benötigt werden und unter strengem Feuchtigkeitsausschluß gearbeitet werden muß (vgl. Ber. dtsch. chem. Ges. 16, 1643 [1883]; Organic Syntheses, Coll. Vol. I, 5 [1951]).

Weiterhin werden in Chem. Abstr. 106: 138 338 e und Chem. Abstr. 90: 203 981 b die Synthese von 1,2,4-Triazolin-5-onen und 4-Amino-3-aryl-1,2,4-triazol-5-onen beschrieben.

Es wurde nun gefunden, daß man 4-Amino-1,2,4-triazol-5-one der allgemeinen Formel (I), 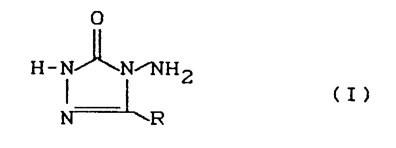
in welcher
- R: für jeweils geradkettiges oder verzweigtes Alkyl oder Alkenyl mit bis zu 8 Kohlenstoffatomen steht, welche jeweils gegebenenfalls durch C₃-C₆-Cycloalkyl, Phenyl, C₁-C₄-Alkoxy, C₁-C₄-Alkylamino, Di(C₁-C₄-alkyl)-amino, Hydroxy, Amino oder Halogen substituiert sind, oder R für Cycloalkenyl oder Cycloalkyl mit jeweils bis zu 6 Kohlenstoffatomen steht, welche jeweils gegebenenfalls durch Hydroxy, Amino, Halogen, Phenyl, C₁-C₄-Alkoxy, C₁-C₄-Alkylamino oder Di-(C₁-C₄-alkyl)-amino substituiert sind, oder R für Benzyl, Phenyl, Pyridyl oder Thienyl steht,
in guten Ausbeuten und in hoher Reinheit erhält,
wenn man Carbodihydrazid der Formel (II)

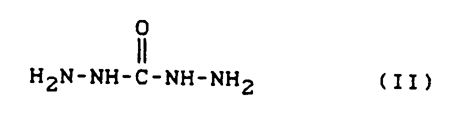

mit Nitrilen der allgemeinen Formel (III)

R-C≡N (III)

in welcher
- R: die oben angegebene Bedeutung hat,
gegebenenfalls in Gegenwart eines Reaktionshilfsmittels und gegebenenfalls in Gegenwart eines Verdünnungsmittels bei Temperaturen zwischen 20°C und 250°C umsetzt.

Das Carbodihydrazid kann bei diesem Verfahren entweder (a) isoliert eingesetzt oder (b) vorgeschaltet erzeugt und in situ weiter umgesetzt werden.

Es ist als ausgesprochen überraschend zu bezeichnen, daß die erfindungsgemäße Umsetzung die Verbindungen der Formel (I) in guten Ausbeuten und in hoher Reinheit liefert, da nach den oben angegebenen bekannten Synthesemethoden entweder teure oder aufwendig herzustellende Ausgangsstoffe einzusetzen sind oder nur unbefriedigende Ausbeuten erreicht werden.

Das erfindungsgemäße Verfahren weist eine Reihe von Vorteilen auf: Die einzusetzenden Ausgangsstoffe der Formeln (II) und (III) sind billige handelsübliche Materialien; ihre Umsetzung und die Aufarbeitung können auf relativ einfache Weise durchgeführt werden. Gegenüber den bekannten Verfahren können im allgemeinen verbesserte Ausbeuten erzielt werden. Schließlich können auch Aminotriazolone hergestellt werden, die nach bekannten Verfahren nicht zugänglich sind.

Ein besonderer Vorteil des erfindungsgemäßen Verfahrens liegt darin, daß das als Ausgangsstoff einzusetzende Carbodihydrazid der Formel (II) nicht nur als isolierte Substanz eingesetzt werden kann, sondern auch alternativ in einer vorgeschalteten Reaktion nach üblichen Methoden aus billigen Ausgangsstoffen erzeugt und in situ, d.h. in einem "Ein-Topf-Verfahren", weiter mit Verbindungen der Formel (III) umgesetzt werden kann.

Die nach dem erfindungsgemäßen Verfahren herzustellenden 4-Amino-1,2,4-triazol-5-one sind durch die Formel (I) allgemein definiert.

Insbesondere werden nach dem erfindungsgemäßen Verfahren Verbindungen der Formel (I) hergestellt,
in welcher
- R: für Methyl, Ethyl, Propyl, Isopropyl, Butyl, Isobutyl, sec-Butyl, tert-Butyl, Pentyl, Isopentyl, sec-Pentyl, tert-Pentyl, Cyclopentenyl, Cyclohexenyl, Cyclopropyl, Cyclobutyl, Cyclopentyl oder Cyclohexyl steht, wobei die genannten Reste gegebenenfalls durch Fluor und/oder Chlor einfach, zweifach oder dreifach substituiert sind, oder R für Benzyl oder Phenyl steht.

Verwendet man beispielsweise Carbodihydrazid und Pivalinsäurenitril als Ausgangsstoffe, so kann der Reaktionsablauf beim erfindungsgemäßen Verfahren durch das folgende Formelschema wiedergegeben werden: 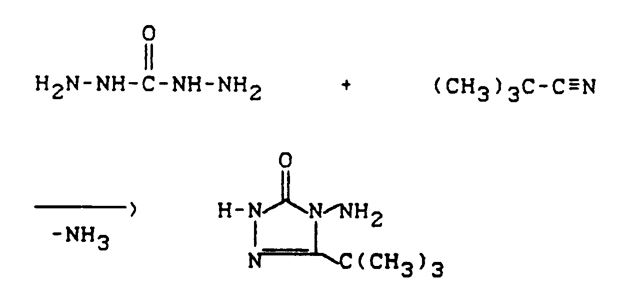
Das beim erfindungsgemäßen Verfahren als Ausgangsstoff zu verwendende Carbodihydrazid der Formel (II) ist eine bekannte, handelsübliche Chemikalie. Auf die mögliche vorgeschaltete Herstellung und in-situ-Umsetzung dieser Verbindung wird weiter unten eingegangen.

Die beim erfindungsgemäßen Verfahren weiter als Ausgangsstoffe zu verwendenden Nitrile sind durch die Formel (III) allgemein definiert. In der Formel (III) hat R vorzugsweise bzw. insbesondere diejenige Bedeutung, die bereits oben im Zusammenhang mit der Beschreibung der Verbindungen der Formel (I) vorzugsweise bzw. als insbesondere bevorzugt für R angegeben wurde.

Als Beispiele für die Ausgangsstoffe der Formel (III) seien genannt:
Acetonitril, Propionitril, Butyronitril, Isobutyronitril, Valeronitril, Isovaleronitril, Cyclopropancarbonsäurenitril, Cyclobutancarbonsäurenitril, Cyclopentancarbonsäurenitril, Cyclohexancarbonsäurenitril, Cyclohexencarbonsäurenitril, Phenylacetonitril, Benzonitril.

Die Ausgangsstoffe der Formel (III) sind bekannte organische Synthesechemikalien.

Das als Ausgangsstoff zu verwendende Carbodihydrazid der Formel (II) kann zur Durchführung des erfindungsgemäßen Verfahrens in einer vorgeschalteten Stufe hergestellt werden.

Hierzu werden Kohlensäurederivate der allgemeinen Formel (IV) 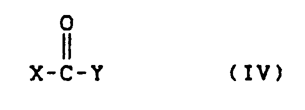
in welcher
- X und Y: für in der Kohlensäurechemie übliche Abgangsgruppen stehen,
mit Hydrazin oder Hydrazinhydrat, gegebenenfalls in Gegenwart eines Verdünnungsmittels und gegebenenfalls in Gegenwart eines Reaktionshilfsmittels, bei Temperaturen zwischen -30°C und +180°C, vorzugsweise zwischen -10°C und +160°C, umgesetzt.

Als geeignete Abgangsgruppen (X, Y) bei den Verbindungen der Formel (IV) seien genannt:
Halogen, vorzugsweise Chlor, Amino, geradkettiges oder verzweigtes Alkoxy, vorzugsweise mit 1 bis 6 Kohlenstoffatomen, insbesondere Methoxy oder Ethoxy, Aryloxy, vorzugsweise Phenoxy.

X und Y können auch zusammen für geradkettiges oder verzweigtes Alkylendioxy, vorzugsweise mit 2 bis 6 Kohlenstoffatomen, insbesondere für Ethylendioxy oder Propylendioxy stehen.

Die Kohlensäurederivate der Formel (IV) können auch Bestandteile von oligomeren oder polymeren Kohlensäurederivaten sein, deren Alkoholkomponente ein Oxaalkandiol, wie z.B. Diethylenglycol ist.

Als Beispiele für die Ausgangsstoffe der Formel (IV) seien genannt:
Phosgen, Harnstoff, Chlorameisensäure-methylester und -ethylester, Dimethyl- und Diethyl-carbonat, Diphenylcarbonat, Carbamidsäure-methylester und -ethylester sowie 2-Oxo-1,3-dioxolan ("Glycolcarbonat") und Propylencarbonat.

Die Ausgangsstoffe der Formel (IV) sind bekannte Synthesechemikalien.

Das erfindungsgemäße Verfahren zur Herstellung von 4-Amino-1,2,4-triazol-5-onen wird - wie auch gegebenenfalls die vorgeschaltete Stufe zur Herstellung von Carbodihydrazid - gegebenenfalls in Gegenwart eines Verdünnungsmittels durchgeführt. Als Verdünnungsmittel kommen dabei neben Wasser praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Mesitylen, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, ferner Dimethylsulfoxid, Tetramethylensulfon, Hexamethylphosphorsäuretriamid und N-Methyl-pyrrolidon; schließlich auch Alkohole wie Methanol, Ethanol, Propanol, Isopropanol, Butanol, Isobutanol, sec-Butanol, tert-Butanol, Pentanol, Isopentanol, sec-Pentanol und tert-Pentanol, Diole wie Ethan-1,2-diol, Propan-1,2-diol und Propan-1,3-diol, Alkoxyalkohole wie Methoxyethanol und Ethoxyethanol, sowie Hydroxyarene wie Phenol. Phenol und die genannten Diole werden als Verdünnungsmittel besonders bevorzugt.

Das erfindungsgemäße Verfahren zur Herstellung von 4-Amino-1,2,4-triazol-5-onen wird - wie auch gegebenenfalls die vorgeschaltete Stufe zur Herstellung von Carbodihydrazid - gegebenenfalls in Gegenwart eines Reaktionshilfsmittels durchgeführt. Als Reaktionshilfsmittel kommen im allgemeinen Metallverbindungen, vorzugsweise Zinnverbindungen in Betracht. Als Beispiele hierfür seien genannt:
Dibutylzinndichlorid, Dimethylzinndichlorid, Dibutylzinnoxid, Hexabutyldistannoxan, Butylstannonsäure, Dibutylzinndilaurat und Dimethylzinnoxid.

Bei Verwendung "aprotischer" Verdünnungsmittel, wie z.B. N-Methyl-pyrrolidon, ist zusätzlich die Verwendung einer "H-aciden" Verbindung, wie z.B. Phenol, Ethylenglycol, Propylenglycol, Diethylamin, Dipropylamin oder Dibutylamin, als weiterem Reaktionshilfsmittel zweckmäßig.

Die Reaktionstemperaturen können bei dem erfindungsgemäßen Verfahren in einem größeren Bereich variiert werden. Im allgemeinen arbeitet man bei Temperaturen zwischen 20 °C und 250 °C, vorzugsweise bei Temperaturen zwischen 60°C und 200 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens setzt man auf 1 Mol Carbodihydrazid der Formel (II) im allgemeinen zwischen 0,5 und 3 Mol, vorzugsweise zwischen 0,8 und 1,5 Mol, Nitril der Formel (III) ein.

In einer ersten bevorzugten Variante zur Durchführung des erfindungsgemäßen Verfahrens wird das Carbodihydrazid der Formel (II) in einem der oben angegebenen Verdünnungsmittel vorgelegt und nach Zugabe eines Nitrils der Formel (III) und gegebenenfalls eines Reaktionshilfsmittels wird die Mischung, vorzugsweise bei erhöhter Temperatur zwischen 60°C und 200°C, gerührt, bis die Ammoniak-Entwicklung praktisch beendet ist.

Zur Isolierung des Reaktionsproduktes der Formel (I) werden die flüchtigen Komponenten unter vermindertem Druck abdestilliert. Die Reinigung des als fester Rückstand anfallenden Produktes erfolgt vorzugsweise durch Umkristallisation.

In einer zweiten bevorzugten Variante des erfindungsgemäßen Verfahrens wird zunächst in einer vorgeschalteten Stufe das Carbodihydrazid der Formel (II) aus einem Kohlensäurederivat der Formel (IV) und Hydrazin oder Hydrazinhydrat erzeugt, vorzugsweise indem man Hydrazin oder Hydrazinhydrat in einem Wasser- oder Eisbad vorlegt und das Kohlensäurederivat der Formel (IV) langsam eindosiert. Das Reaktionsgemisch wird dann im allgemeinen mehrere Stunden bei erhöhter Temperatur gerührt. Dann werden gegebenenfalls flüchtige Komponenten (wie z.B. Wasser aus Hydrazinhydrat) unter vermindertem Druck abdestilliert und das im Rückstand verbleibende Carbodihydrazid der Formel (II) wie oben beschrieben mit einem Nitril der Formel (III) umgesetzt.

Die nach dem erfindungsgemäßen Verfahren herzustellenden 4-Amino-1,2,4-triazol-5-one der Formel (I) können als Zwischenprodukte zur Herstellung von Herbiziden verwendet werden (vgl. US-P 3884910 oder EP-A 294666).

### Herstellungsbeispiele:

### Beispiel 1 / Verbindung-Nr. I-1

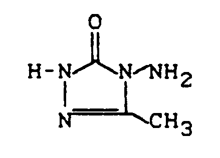

180 g (2,0 Mol) Carbodihydrazid werden in 360 g Ethylenglycol suspendiert und bei 20°C bis 30°C werden unter Rühren 90 g (2,2 Mol) Acetonitril und 0,5 g Dibutylzinnoxid dazugegeben. Das Reaktionsgemisch wird dann zum schwachen Rückfluß (102°C) erhitzt und im Verlauf von 8 Stunden wird die Rückflußtemperatur bis auf 170°C gesteigert. Die Ammoniak-Entwicklung ist dann praktisch beendet. Anschließend wird unter vermindertem Druck (zuletzt bei 1 mbar) zur Trockne eingeengt und der Rückstand aus 250 ml Wasser umkristallisiert. Man erhält 176 g (77% der Theorie) 3-Methyl-4-amino-1,2,4-triazol-5-on vom Schmelzpunkt 225°C.

### Beispiel 2 / Verbindung-Nr. I-1

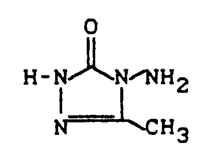

Eine Mischung aus 180 g (2,0 Mol) Carbodihydrazid, 360 g N-Methyl-pyrrolidon, 90 g (2,2 Mol) Acetonitril und 0,5 Dibutylzinnoxid wird zum leichten Rückfluß (122°C) erhitzt. Dann werden 65 g Dibutylamin dazugegeben und im Verlauf von 6 Stunden wird die Rückflußtemperatur auf 160°C gesteigert, wonach die Ammoniak-Entwicklung praktisch beendet ist. Nach Abdestillieren der flüchtigen Bestandteile, zuletzt bei 1 mbar, wird der Rückstand aus 250 ml Wasser umkristallisiert. Man erhält 150 g (67% der Theorie) 3-Methyl-4-amino-1,2,4-triazol-5-on vom Schmelzpunkt 228°C.

### Beispiel 3 / Verbindung-Nr. I-1

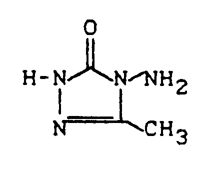

### Eintopf-Verfahren

In einem 61-Vierhalskolben mit Kontaktthermometer, Rührer, Tropftrichter, kurzer Kolonne und Rücklaufteiler werden 1354 g (27,1 Mol) Hydrazinhydrat unter Eiskühlung vorgelegt. Im Verlauf von 2 Stunden werden 2898 g (13,5 Mol) Diphenylcarbonat in fester Form so eingetragen, daß durch die sofort einsetzende exotherme Reaktion die Temperatur nicht über 40°C ansteigt. Dann wird das Kühlbad entfernt und noch 3 Stunden bei 80°C nachgerührt. Danach ist die Menge unreagierten Hydrazins unter 0,4 Mol (bestimmt durch Titration gegen HCl) abgesunken. Nun wird unter vermindertem Druck das Hydratwasser und bei vollem Wasserstrahlvakuum noch 428 g Phenol abgezogen (Sumpftemperatur etwa 105°C). Nach Aufheben des Vakuums mit Stickstoff werden über den Tropftrichter bei gleicher Temperatur 610 g (14,9 Mol) Acetonitril eingerührt, worauf ein Teil des gebildeten Carbodihydrazids ausfällt, der Ansatz aber noch gut rührbar bleibt. Durch schwache Wärmezufuhr wird die Temperatur allmählich gesteigert, bei einer Sumpftemperatur von 118°C setzt Acetonitril-Rückfluß (Einstellung des Rücklaufteilers auf vollen Rücklauf) ein; schwache NH₃-Entwicklung beginnt. Der Gasstrom wird über eine Sicherheitswaschflasche und ein Rückschlagventil in vorgelegte Portionen verdünnter Schwefelsäure (Indikator Bromphenolblau) geleitet, und so der Fortgang der Reaktion kontrolliert. Im Verlauf von 18 Stunden steigt durch Acetonitril-Abnahme die Sumpftemperatur auf 136°C, die NH₃-Entwicklung wird zunehmend zügiger. Nach weiteren 3 Stunden (Sumpftemperatur nun 150°C) ist die Hälfte der theoretischen Menge Ammoniak abgespalten. Die Temperatur wird weiter gesteigert, nach weiteren 4 Stunden (Temp. 170°C) sind 80% NH₃ freigesetzt. Der Ansatz wird noch 6 Stunden bei 180°C gerührt, die NH₃-Menge beträgt dann 98% der Theorie. Nun werden unter vermindertem Druck zunächst niedrig siedende Anteile und dann Phenol abdestilliert.

Der Druck wird so eingestellt, daß bei zügiger Destillation die Sumpftemperatur ständig 160°C-180°C beträgt. So wird ein vorzeitiges Auskristallisieren des Endprodukts vermieden. Wenn 80-85% des Phenols abdestilliert sind, werden Heizung und Rührer abgestellt und der Druck zügig auf 15 mbar erniedrigt, wobei restliches Phenol abdestilliert und der Ansatz kristallin erstarrt. Nach Abkühlen wird der Kolbeninhalt durch Aufkochen mit 1800 ml Wasser in Lösung gebracht.

Nun werden durch Abdestillieren von 350 ml Wasser geringe Restmengen von Phenol abgetrieben, bei 80°C 82 g (1,1 Mol) Diethylamin eindosiert, durch Andestillieren überschüssiges Diethylamin (0,4 Mol) abgetrennt und die klare gelbliche Lösung in ein Becherglas ausgegossen. Nach Abkühlen auf 10°C wird das auskristallisierte Aminotriazolon abgesaugt, mit 500 ml kaltem, wäßrigem Ethanol gewaschen und bei 100°C im Vakuum getrocknet. Man erhält 1102 g (71% der Theorie) 3-Methyl-4-amino-1,2,4-triazol-5-on vom Schmelzpunkt 230°C.

### Beispiel 4 / Verbindung-Nr. I-1

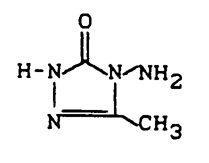

### Eintopfverfahren

1270 g Phenol werden auf 80°C erwärmt und 810 g (13,5 Mol) Harnstoff und 1350 g (27 Mol) Hydrazinhydrat dazugegeben. Beim weiteren Erwärmen des Gemisches wird ab 110°C zügig Ammoniak abgespalten. Im Verlauf von 4 Stunden wird die Temperatur auf 125°C gesteigert; es werden 13,5 Mol Ammoniak in verdünnter Schwefelsäure aufgefangen. Danach kommt die Gasentwicklung zum Erliegen, weil mit dem rückfließenden Wasser auch der Ammoniak zurückgeführt wird, und so ein Gleichgewicht entsteht. Nun werden unter 50% Rücklauf Wasser und Ammoniak abdestilliert und die Temperatur stetig erhöht. Im Verlauf von 5 Stunden wird eine Sumpftemperatur von 150°C erreicht, dabei sind 800 g abdestilliert, die 12,15 Mol Ammoniak, 24,3 Mol Wasser und 3 Mol Hydrazin enthalten. Nun werden 3,5 Mol Hydrazin nachgesetzt und auf 140°C aufgeheizt. Durch Zutropfen von 554 g (13,5 Mol) Acetonitril (Anfangsmenge ca. 100 ml) wird bei 130-140°C stetiger Rückfluß aufrecht erhalten Innerhalb 7 Stunden kann so alles Acetonitril zugegeben werden; es entstehen dabei 8 Mol NH₃. Im Verlauf von weiteren 7 Stunden werden unter Rückfluß 160°C erreicht, die Gesamtmenge an abgespaltenem Ammoniak erhöht sich auf 39 Mol (96,3% d.Th.). Nun wird Phenol wie in Bsp. 3 abdestilliert und der Rückstand in 1750 ml Wasser gelöst. Durch Andestillieren werden Spuren von Phenol entfernt. Nach Abkühlen auf 10°C wird abgesaugt, mit kaltem 50%igem Ethanol gewaschen und getrocknet. Man erhält 926 g (60% der Theorie) 3-Methyl-4-amino-1,2,4-triazol-5-on vom Schmelzpunkt 230°C.

### Beispiel 5 / Verbindung-Nr. I-1

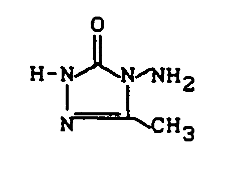

Eine Mischung aus 1175 g Phenol, 1113 g (12,5 Mol) Carbamidsäurethylester und 1250 g (25 Mol) Hydrazinhydrat wird zum Rückfluß erwärmt (Sumpftemperatur 117°C). In 90 Minuten werden dabei 3 Mol Ammoniak freigesetzt; danach kommt die Gasentwicklung zum Erliegen. Nun wird bei einem Rücklauf von 75% ein Gemisch aus Alkohol, Wasser, Ammoniak und Hydrazin abdestilliert, wobei die Temperatur stetig gesteigert wird. Nach 6 Stunden sind 160°C erreicht. Es sind dann ca. 1100 g abdestilliert, die 22,5 Mol (90% d.Th.) Wasser, 10 Mol (80% d.Th.) Ethanol, 7 Mol (56% d.Th.) Ammoniak und 2 Mol Hydrazin enthalten.

Nun werden 2 Mol Hydrazinhydrat bei 80°C nachgesetzt und 513 g (12,5 Mol) Acetonitril zugegeben und unter schwachem Rückfluß gerührt. Nach 7 Stunden (Sumpftemp. 140°C) werden weitere 102 g Acetonitril (2,5 Mol) zugegeben. Nach 15 Stunden ist eine Endtemperatur von 160°C erreicht. Die NH₃-Entwicklung kommt zum Erliegen. Es wird wie in Bsp. 4 aufgearbeitet. Man erhält 880 g (62% der Theorie) 3-Methyl-4-amino-1,2,4-triazol-5-on vom Schmelzpunkt 230°C.

### Beispiel 6 / Verbindung-Nr. I-1

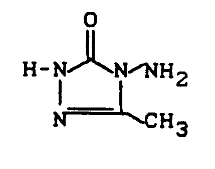

### Eintopfverfahren

Eine Mischung aus 1650 g Phenol, 1150 g (23 Mol) Hydrazinhydrat und 1298 g (11 Mol) Diethylcarbonat wird 30 Minuten unter Rückfluß erhitzt. Dann wird bis zu einer Sumpftemperatur von 160°C bei einem Rücklauf von 75% Alkohol, Wasser und 1,5 Mol Hydrazin abdestilliert. Nach Abkühlen auf 80°C werden 1 g Dibutylzinnoxid und 451 g (11 Mol) Acetonitril zugefügt und bis zum Rückfluß (120°C) erhitzt. Im Verlauf von 8 Stunden kann die Temperatur durch die Abnahme des Acetonitrilgehalts bedingt auf 160°C gesteigert werden. Danach ist die Gasentwicklung beendet. Nach Abdestillieren von geringen Mengen Wasser, Alkohol und Acetonitril wird wie in Bsp. 3 Phenol abdestilliert und der Rückstand aus 900 ml H₂O umkristallisiert. Man erhält 770 g (61% der Theorie) 3-Methyl-4-amino-1,2,4-triazol-5-on vom Schmelzpunkt 230°C.

### Beispiel 7 / Verbindung-Nr I-2

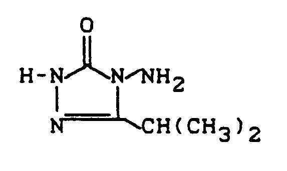

Analog Beispiel 3 wird aus 2264 g (10,6 Mol) Diphenylcarbonat und 1058 g (21,2 Mol) Hydrazinhydrat eine Lösung von Carbodihydrazid in Phenol hergestellt und unter vermindertem Druck weitgehend entwässert. Nach Zugabe von 1 g Dimethylzinnoxid und 803 g (11,6 Mol) Isobutyronitril wird 10 Stunden unter Rückfluß (Anfangstemperatur 138°C, Endtemperatur 185°C) erhitzt. Dann werden die flüchtigen Komponenten unter vermindertem Druck abdestilliert und der Rückstand wird in 1,5 l Wasser heiß gelöst. Bei 80°C werden 116 g (1,6 Mol) Diethylamin dazugegeben und dann nicht verbrauchtes Amin und restliches Phenol abdestilliert. Nach Kühlen auf 10°C wird abgesaugt, mit kaltem Wasser gewaschen und getrocknet. Man erhält 1129 g (75% der Theorie) 3-Isopropyl-4-amino-1, 2,4-triazol-5-on vom Schmelzpunkt 172°C.

### Beispiel 8 / Verbindung-Nr. I-3

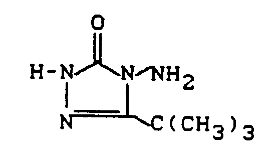

Aus 1900 g (38 Mol) Hydrazinhydrat und 4066 g (19 Mol) Diphenylcarbonat wird analog Bsp. 3 eine Lösung von Carbodihydrazid in Phenol hergestellt und vom Hydratwasser befreit. Im Anschluß an das Wasser werden zur besseren Raumausnutzung noch 1600 ml Phenol i.Vak. abdestilliert. Das Vakuum wird bei 80°C mit Stickstoff aufgehoben und dann 30 g Dibutylzinndichlorid und 1577 g (19 Mol) Pivalonitril zugefügt. Beim Aufheizen beginnt bei 125°C zügige NH₃-Entwicklung. Nach 5 Stunden Rückfluß werden weitere 158 g (1,9 Mol) Pivalonitril zugegeben und innerhalb 4 Stunden bis 180°C aufgeheizt. Danach ist die Gasentwicklung beendet. Es wird nun analog Bsp. 3 bis zur Trockne eingedampft und der Rückstand in 3 l Dimethylformamid heiß gelöst. Nach Abkühlen auf 10°C Absaugen, Waschen mit kaltem Wasser und Trocknen bei 150°C i.Vak. werden 1703 g (57,4% d.Th.) des Triazolons vom Schmelzpunkt 248°C erhalten. Durch Eingießen der Mutterlauge in Eiswasser und Isolierung der entstandenen Fällung werden weitere 320 g (10,8%. d.Th.) gewonnen. Gesamtausbeute: 2023 g (68% d.Th.) 3-tert-Butyl-4-amino-1,2,4-triazol-5-on.

### Beispiel 9 / Verbindung-Nr. I-4

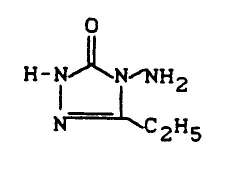

Aus 3200 g (64 Mol) Hydrazinhydrat und 6848 g (32 Mol) Diphenylcarbonat wird analog Beispiel 3 eine Lösung von Carbodihydrazid in Phenol hergestellt und anschließend das Hydratwasser und noch 1500 ml Phenol abdestilliert. Nach Zufügen von 3 g Dimethylzinnoxid und 1848 g (33,6 Mol) Propionitril wird bis zum schwachen Rückfluß erhitzt (125°C). Innerhalb von 11 Stunden kann unter ständigem Rückfluß die Temperatur auf 180°C gesteigert werden. Danach ist die NH₃-Entwicklung beendet. Nun wird Phenol bei einer Sumpftemperatur von 160°C bei vermindertem Druck (abnehmend bis 15 mbar) abdestilliert, wobei der Rückstand flüssig bleibt. Restliches Phenol wird deshalb an der Ölpumpe bei 1 mbar entfernt. Nach Abkühlen wird der Rückstand in 3 l heißem Wasser gelöst. Die Lösung wird mit 80 ml Diethylamin und 10 g Weinsäure (zur Beseitigung einer schwachen, durch den Katalysator verursachten Trübung) versetzt. Durch Andestillieren wird ein Diethylamin-Überschuß und Spuren von Phenol entfernt. Nach Abkühlen auf 10°C wird abgesaugt, mit 500 ml kaltem 50%igem Ethanol gewaschen und bei 100°C im Vakuum getrocknet. Man erhält 3478 g (85% der Theorie) 3-Ethyl-4-amino-1,2,4-triazol-5-on vom Schmelzpunkt 171°C.

### Beispiel 10 / Verbindung-Nr. I-5

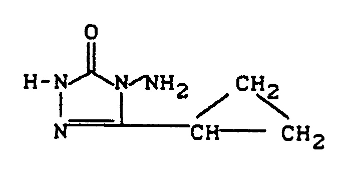

Aus 1100 g (22 Mol) Hydrazinhydrat und 2354 g (11 Mol) Diphenylcarbonat wird analog Beispiel 3 eine Lösung von Carbodihydrazid in Phenol hergestellt und entwässert. Bei 80°C werden 0,5 g Dibutylzinnoxid, 774 g (11,5 Mol) Cyclopropylcyanid und 100 ml Petrolether (Siedebereich 80°C-90°C) dazugegeben. Das Gemisch wird 5 Stunden zum Rückfluß erhitzt (Anfangstemperatur: 130°C, Endtemperatur 170°C). Flüchtige Komponenten werden im Vakuum abdestilliert und der Rückstand in 2 l Wasser gelöst. Nach Zugabe von 38 ml Diethylamin und 5 g Weinsäure wird andestilliert, und nach Abkühlen abgesaugt. Man erhält 1172 g (80% der Theorie) 3-Cyclopropyl-4-amino-1,2,4-triazol-5-on vom Schmelzpunkt 183°C.

### Beispiel 11 / Verbindung-Nr. I-1

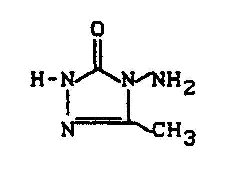

### Eintopfverfahren

400 g (8 Mol) Hydrazinhydrat werden unter Wasserkühlung zu einer Mischung aus 704 g (8 Mol) Glycolcarbonat und 300 g Ethylenglycol gegeben und das Gemisch wird 60 Minuten bei 50°C gerührt. Dann werden im Wasserstrahlvakuum 131 g Wasser abdestilliert. Nach Zugabe von weiteren 400 g (8 Mol) Hydrazinhydrat, 361 g (8,8 Mol) Acetonitril und 1 g Dibutylzinnoxid wird das Gemisch 60 Stunden unter Rückfluß erhitzt und anschließend wie in Beispiel 10 aufgearbeitet. Man erhält 574 g (63%. der Theorie) 3-Methyl-4-amino-1,2,4-triazol-5-on vom Schmelzpunkt 231°C.

### Beispiel 12 / Verbindung-Nr. I-1

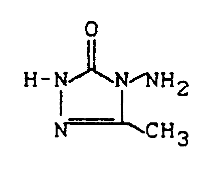

### Eintopfverfahren

Aus 1300 g (26 Mol) Hydrazinhydrat und 2782 (13 Mol) Diphenylcarbonat wird analog Beispiel 3 eine Lösung von Carbodihydrazid in Phenol hergestellt und entwässert. Nach Zugabe von 560 g (13,65 Mol) Acetonitril und 2 g Dibutylzinnoxid wird das Gemisch 70 Stunden bei 100°C gerührt und anschließend schnell auf 160°C aufgeheizt. Nach Aufarbeitung analog Beispiel 3 erhält man 1126 g (76% der Theorie) 3-Methyl-4-amino-1,2,4-triazol-5-on vom Schmelzpunkt 231°C.

## Patentansprüche

1. Verfahren zur Herstellung von 4-Amino-1,2,4-triazol-5-onen der allgemeinen Formel (I), 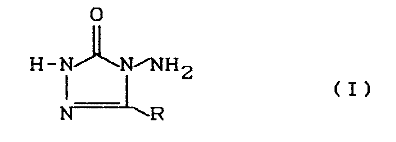 in welcher
R für jeweils geradkettiges oder verzweigtes Alkyl oder Alkenyl mit bis zu 8 Kohlenstoffatomen steht, welche jeweils gegebenenfalls durch C₃-C₆-Cycloalkyl, Phenyl, C₁-C₄-Alkoxy, C₁-C₄-Alkylamino, Di- (C₁-C₄-alkyl)-amino, Hydroxy, Amino oder Halogen substituiert sind, oder R für Cycloalkenyl oder Cycloalkyl mit jeweils bis zu 6 Kohlenstoffatomen steht, welche jeweils gegebenenfalls durch Hydroxy, Amino, Halogen, Phenyl, C₁-C₄-Alkoxy, C₁-C₄-Alkylamino oder Di-(C₁-C₄-alkyl)-amino substituiert sind, oder R für Benzyl, Phenyl, Pyridyl oder Thienyl steht.
dadurch gekennzeichnet, daß man Carbodihydrazid der Formel (II) 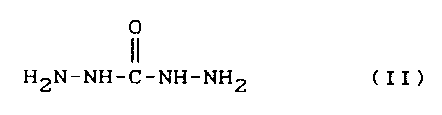 mit Nitrilen der allgemeinen Formel (III)
R-C≡N (III)
in welcher
R die oben angegebene Bedeutung hat,
gegebenenfalls in Gegenwart eines Reaktionshilfsmittels und gegebenenfalls in Gegenwart eines Verdünnungsmittels bei Temperaturen zwischen 20°C und 250°C umsetzt.

2. Verfahren gemäß Anspruch 1, dadurch gekennzeichnet, daß man das Carbodihydrazid der Formel (II) vorgeschaltet erzeugt, wobei man Kohlensäurederivate der Formel (IV) 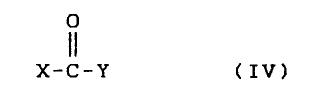 in welcher
X und Y jeweils für eine Abgangsgruppe stehen,
mit Hydrazin oder Hydrazinhydrat umsetzt und in situ weiter mit Verbindungen der Formel (III) umsetzt.

3. Verfahren gemäß Anspruch 1, wobei als Reaktionshilfsmittel Metallverbindungen, insbesondere Dibutylzinndichlorid, Dimethylzinndichlorid, Dibutylzinnoxid, Hexabutyldistannoxan, Butylstannonsäure, Dibutylzinndilaurat und Dimethylzinnoxid, verwendet werden.

4. Verfahren gemäß Anspruch 1, wobei bei Verwendung aprotischer Verdünnungsmittel zusätzlich H-acide Verbindungen als Reaktionshilfsmittel verwendet werden können.

5. Verfahren gemäß Anspruch 1, dadurch gekennzeichnet, daß auf 1 Mol Carbodihydrazid der Formel (II) zwischen 0,5 und 3 Mol Nitril der Formel (III) eingesetzt wird.

6. Verfahren gemäß Anspruch 1, dadurch gekennzeichnet, daß man es als Ein-Topf-Verfahren durchführt.

## Claims

1. Process for the preparation of 4-amino-1,2,4-triazol-5-ones of the general formula (I) 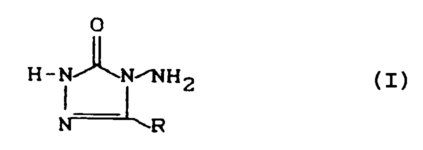 in which
R represents in each case straight-chain or branched alkyl or alkenyl having up to 8 carbon atoms, in each case optionally substituted by C₃-C₆-cycloalkyl, phenyl, C₁-C₄-alkoxy, C₁-C₄-alkylamino, di-(C₁-C₄-alkyl)-amino, hydroxyl, amino or halogen, or R represents cycloalkenyl or cycloalkyl having in each case up to 6 carbon atoms, in each case optionally substituted by hydroxyl, amino, halogen, phenyl, C₁-C₄-alkoxy, C₁-C₄-alkylamino or di-(C₁-C₄-alkyl)-amino, or R represents benzyl, phenyl, pyridyl or thienyl,
characterized in that carbodihydrazide of the formula (II) 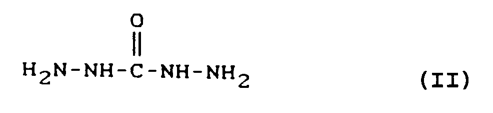 is reacted with nitriles of the general formula (III)
R-C≡N (III)
in which
R has the abovementioned meaning,
if appropriate in the presence of a reaction auxiliary and if appropriate in the presence of a diluent at temperatures between 20°C and 250°C.

2. Process according to Claim 1, characterized in that the carbodihydrazide of the formula (II) is produced in a preliminary stage in which carbonic acid derivatives of the formula (IV) 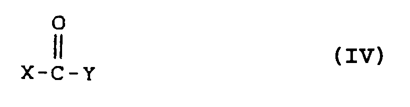 in which
X and Y in each case represent a leaving group,
are reacted with hydrazine or hydrazine hydrate and the product is further reacted with compounds of the formula (III) in situ.

3. Process according to Claim 1, wherein metal compounds, in particular dibutyltin dichloride, dimethyltin dichloride, dibutyltin oxide, hexabutyldistannoxane, butylstannonic acid, dibutyltin dilaurate and dimethyltin oxide, are used as reaction auxiliaries.

4. Process according to Claim 1, wherein H-acid compounds can additionally be used as reaction auxiliaries if aprotic diluents are used.

5. Process according to Claim 1, characterized in that between 0.5 and 3 mol of nitrile of the formula (III) are employed per mol of carbodihydrazide of the formula (II).

6. Process according to Claim 1, characterized in that it is carried out as a one-pot process.

## Revendications

1. Procédé de production de 4-amino-1,2,4-triazole-5-ones de formule générale (I), 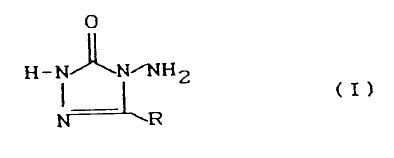 dans laquelle
R désigne un groupe alkyle ou un groupe alcényle linéaire ou ramifié ayant chacun jusqu'à 8 atomes de carbone, ces groupes étant substitués chacun le cas échéant par un radical cycloalkyle en C₃ à C₆, phényle, alkoxy en C₁ à C₄, alkylamino en C₁ à C₄, di(alkyle en C₁ à C₄)amino, hydroxy, amino ou halogéno, ou bien R est un groupe cycloalcényle ou groupe cycloalkyle, ayant chacun jusqu'à 6 atomes de carbone, qui sont substitués chacun le cas échéant par un radical hydroxy, amino, halogéno, phényle, alkoxy en C₁ à C₄, alkylamino en C₁ à C₄, di(alkyle en C₁ à C₄)amino, ou bien R est un groupe benzyle, phényle, pyridyle ou thiényle,
caractérisé en ce qu'on fait réagir le carbodihydrazide de formule (II) 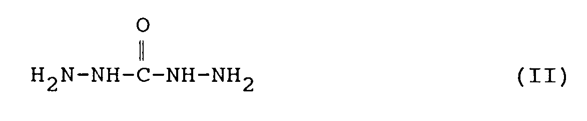 avec des nitriles de formule générale (III)
R-C-≡N (III)
dans laquelle
R a la définition indiquée ci-dessus,
le cas échéant en présence d'une substance auxiliaire de réaction et en la présence éventuelle d'un diluant, à des températures comprises entre 20°C et 250°C.

2. Procédé suivant la revendication 1, caractérisé en ce que le carbodihydrazide de formule (II) est produit au préalable, par réaction de dérivés d'acide carbonique de formule (IV) 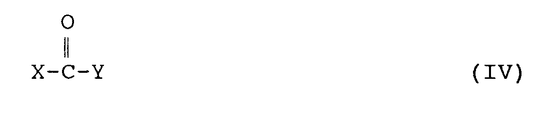 dans laquelle
X et Y représentent chacun un groupe partant,
avec l'hydrazine ou l'hydrate d'hydrazine et le produit de réaction est ensuite amené à réagir in situ avec des composés de formule (III).

3. Procédé suivant la revendication 1, dans lequel on utilise comme substances auxiliaires de réaction des composés métalliques, notamment du dichlorure de dibutylétain, du dichlorure de diméthylétain, de l'oxyde de dibutylétain, de l'hexabutyldistannoxane, de l'acide butyl stannonique, du dilaurate de dibutylétain et de l'oxyde de diméthylétain.

4. Procédé suivant la revendication 1, dans lequel, lorsqu'on utilise des diluants aprotiques, on peut utiliser en outre des composés H-acides comme substances auxiliaires de réaction.

5. Procédé suivant la revendication 1, caractérisé en ce qu'on utilise, par mole de carbodihydrazide de formule (II), entre 0,5 à 3 moles de nitrile de formule (III).

6. Procédé suivant la revendication 1, caractérisé en ce qu'il est mis en oeuvre comme procédé en récipient unique.
